# EUROPEAN PATENT APPLICATION

(11) **EP 3 343 222 A1**
(43) Date of publication of application: **04.07.2018**
(21) Application number: 16839244.7
(22) Date of filing: 22.08.2016
(51) Int. Cl.: G01N 33/53, G01N 33/92

(54) **METHOD FOR ASSISTING DETERMINATION OF TYPE III HYPERLIPIDEMIA**

(30) Priority: 26.08.2015 JP 2015166764
(71) Applicant: Osaka University, Suita-shi, Osaka 565-0871 (JP); Fujirebio Inc., Tokyo 163-0410 (JP)
(72) Inventor: YAMASHITA, Shizuya, Suita-shi Osaka 565-0871 (JP); MASUDA, Daisaku, Suita-shi Osaka 565-0871 (JP); ISOMURA, Mitsuo, Tokyo 163-0410 (JP)
(74) Representative: Keirstead, Tanis Evelyne
(86) International application number: PCT/JP2016/074402
(87) International publication number: WO 2017/033897

(57) **Abstract**

Disclosed is a method of distinguishing type III hyperlipidemia in the WHO-classified hyperlipidemia types (hereinafter referred to as type III) from other types of hyperlipidemia, which is a simpler method and has a higher diagnostic efficiency than conventional methods. The method of assisting the determination of type III hyperlipidemia includes the steps of: measuring the apoB-48 value, TG value and LDL-C value in a blood sample isolated from a living body; and calculating a ratio of the apoB-48 value divided by the TG value and a ratio of the apoB-48 value divided by the LDL-C value. In cases where both the ratio of the apoB-48 value divided by the TG value and the ratio of the apoB-48 value divided by the LDL-C value are higher than the respective cut-off values, it indicates that the living body is likely to have type III hyperlipidemia.

## Description

### Technical Field

The present invention relates to a method of assisting the determination of type III hyperlipidemia (familial type III hyperlipoproteinemia) which is one of the phenotypes of hyperlipidemia according to the WHO classification.

### Background Art

Hyperlipidemia is a category of dyslipidemia and indicates a condition where serum lipids such as cholesterol, phospholipid and free fatty acid are increased. Dyslipidemia is a disease which leads to, if untreated, development of arteriosclerosis, and furthermore, with the progress of symptoms, leads to development of myocardial infarction and/or cerebral infarction, and to death in the worst case. The establishment of efficient methods for diagnosing and treating the disease has been long-awaited. According to the results from the National Health and Nutrition Survey in Japan, 2010, conducted by Ministry of Health, Labor and Welfare, the ratio of individuals suspected to have dyslipidemia in randomly chosen Japanese nationals with 30 or more years of age has been reported to be 22.3% in men and 17.7% in women.

According to the "Japan Arteriosclerosis Society Guidelines for Prevention of Arteriosclerotic Cardiovascular Disease 2012" (hereinafter also referred to simply as "the guidelines") edited by Japan Arteriosclerosis Society, the criteria for dyslipidemia include ≥140 mg/dL of serum low density lipoprotein (LDL) - cholesterol (LDL-C), ≥150 mg/dL of serum triglyceride (TG), and <40 mg/dL of serum high density lipoprotein (HDL) - cholesterol (HDL-C).

Chylomicron (CM), an intermediate metabolite of CM in blood called chylomicron remnant (CM remnant), very low density lipoprotein (VLDL), an intermediate metabolite of VLDL in blood called very low density lipoprotein remnant (VLDL remnant), low density lipoprotein (LDL), high density lipoprotein (HDL), and the like are known to be representative examples of lipoproteins involved in dyslipidemia.

Among lipoproteins present in blood circulating through the body, the increase of various lipoproteins that promote the deposition of cholesterol on the blood vessel wall, or the decrease of various lipoproteins that prevent the deposition of cholesterol is cited as one of the causes of arteriosclerosis. Thus, there is a need to directly measure the amounts of these lipoproteins in blood to use them for the diagnosis and treatment of dyslipidemia and/or arteriosclerosis.

From the aspect of the lipid metabolism, for example, CM remnant and VLDL remnant (these are called remnant-like lipoproteins (remnant-like particles; RLPs)), and LDL and the like are lipoproteins that deliver cholesterol to the blood vessel wall, while HDL is a lipoprotein that removes cholesterol from arteriosclerotic plaques. RLPs have been indicated to be involved in the formation of the arteriosclerotic lesion in postprandial dyslipidemia and are one of the risk factors for arteriosclerosis. Remnant proteins (apoproteins) constituting RLPs include apoB-48 contained in exogenous CMs or CM remnants, and apoB-100 contained in endogenous VLDLs or VLDL remnants.

ApoB-48 is a peptide having an amino acid sequence identical to a partial amino acid sequence of apoB-100. The amino acid sequence of apoB-48 as well as the amino acid sequence apoB-100 has been reported in Non-Patent Document 1. Anti-apoB-48 antisera have been reported in Non-Patent Documents 2 to 4. Moreover, anti-apoB-48 monoclonal antibodies and methods for measuring apoB-48 in blood by using the same have been reported in Patent Documents 1 and 2, and a standard solution for generating a standard curve, which allows the quantitative measurement of apoB-48, has been reported in Patent Document 3.

According to the WHO classification, hyperlipidemia is classified into the phenotypes I, IIa, IIb, III, IV, and V ("Diagnosis Related Group-based Guidelines for Clinical Examination 2003" edited by Japanese Society of Laboratory Medicine, pp. 118-121). Hyperlipidemia type I is hyperchylomicronemia, type IIa is hypercholesterolemia or hyper-LDL-cholesterolemia, type IIb is a combination of hypercholesterolemia and hypertriglyceridemia, type IV is hypertriglyceridemia, and type V is a combination of hyperchylomicronemia and hyper-VLDL-cholesterolemia. In hyperlipidemia patients, hyperlipidemia type IIa, type IIb, and type IV are often seen.

Type III hyperlipidemia (familial type III hyperlipoproteinemia) is a combination of hypertriglyceridemia and hyper-LDL-cholesterolemia, which is known to be a phenotype associated with a high risk for arteriosclerosis and, however, highly responsive to diet therapy in the early stage, so that early diagnosis and treatment are very useful.

However, because a method of determining the phenotype of hyperlipidemia conventionally depends on electrophoresis, which causes complications associated with the operation, low throughput, and difficulties in objective determination due to its characteristics as a qualitative analysis, it has not been a routinely used method.

Moreover, it is described in Non-Patent Document 5 that the measured value of apoB-48 in blood is usefully used in determination of type III hyperlipidemia using the ratio of the apoB-48 value divided by the TG value. However, although the ratio of apoB-48/TG in type III was demonstrated to be significantly higher than those in the other phenotypes, there remained room for improvement of the diagnostic efficiency with respect to the clinical determination of type III hyperlipidemia.

### Prior Art Documents

### Patent Documents

Patent Document 1: JP 3440852 B
Patent Document 2: JP 3833183 B
Patent Document 3: JP 2013-224863 A

### Non-Patent Documents

Non-Patent Document 1: Nature, Vol. 323, p.738 (October, 1986)
Non-Patent Document 2: J. Biol. Chem., Vol. 265, No. 15, pp. 8358-8360 (1990)
Non-Patent Document 3: J. Biol. Chem., Vol. 267, No. 2, pp. 1175-1182 (1992)
Non-Patent Document 4: Clinical Science, Vol. 85, pp. 521-524 (1993)
Non-Patent Document 5: J. Atheroscler. Thromb., Vol. 19, No. 9, pp. 862-871 (2012)

### Summary of Invention

### Problem to be Solved by the Invention

An object of the present invention is to provide a method of distinguishing type III hyperlipidemia in the WHO-classified hyperlipidemia types (hereinafter referred to as type III) from other types of hyperlipidemia, which is a simpler method and has a higher diagnostic efficiency than conventional methods.

### Means for Solving the Problem

The inventors intensively studied and consequently found that, in the determination of type III hyperlipidemia, the measurement of the apoB-48 value in blood combined not only with the TG value in blood but also with the LDL-C value in blood allows the determination of type III hyperlipidemia with high diagnostic efficiency.

That is, the present invention provides a method of assisting determination of type III hyperlipidemia, the method comprising the steps of: determining the apoB-48 value, TG value and LDL-C value in a blood sample isolated from a living body; and calculating a ratio of the apoB-48 value divided by the TG value and a ratio of the apoB-48 value divided by the LDL-C value, wherein the fact that both the ratio of the apoB-48 value divided by the TG value and the ratio of the apoB-48 value divided by the LDL-C value are higher than the respective cut-off values indicates that the living body is likely to have type III hyperlipidemia.

### Effects of the Invention

According to the method of the present invention, type III hyperlipidemia can be distinguished from other types of hyperlipidemia by a simpler method having a higher diagnostic efficiency than conventional methods.

### Brief Description of the Drawings

Figure 1 shows an ROC curve for apoB-48 (where types I, III, and V are classified as positive) generated in Comparative Example 1 below.
Figure 2 shows an ROC curve for apoB-48 (where type III is classified as positive) generated in Comparative Example 2 below.
Figure 3 shows an ROC curve for apoB-48/TG generated in Comparative Example 3 below.
Figure 4 shows an ROC curve for apoB-48/LDL-C generated in Comparative Example 4 below.

### Detailed Description of the Invention

As described above, in the method of the present invention, the apoB-48 value, TG value and LDL-C value in a blood sample separated from a living body are measured. In this specification, unless otherwise stated, values of apoB-48, TG, and LDL-C are, but not limited to, values expressed in units of µg/mL, mg/dL, and mg/dL, respectively.
Herein, serum or plasma, particularly serum may preferably be used as the blood sample. Additionally, although a TG value and an LDL-C value are measured at a routine health examination and those values can be used, the above three values are preferably measured with a single blood sample for enhancing the accuracy of the determination with excluding changes over time, such as the influence of eating. Moreover, the "living body" which is the source of the blood sample provided for the examination is usually a mammal, and is preferably human. Moreover, a subject who requires the type of hyperlipidemia to be determined is usually a patient with dyslipidemia. The human patient with dyslipidemia is a patient having ≥140 mg/dL of serum low density lipoprotein (LDL) - cholesterol (LDL-C), ≥150 mg/dL of serum triglyceride (TG), or <40 mg/dL of serum high density lipoprotein (HDL) - cholesterol (HDL-C).

The method of measuring the apoB-48 value, the TG value, and the LDL-C value is *per se* well known and kits for that purpose are commercially available and, therefore, these values can be measured by a well-known method using a commercially available kit. A brief explanation is given below.

### <Measurement method for apoB-48>

The measurement method for the apoB-48 value in blood is not particularly limited as long as it is a method that enables quantitative measurement of apoB-48, and an immunoassay using an antibody specific to apoB-48 may preferably be used, and, for example, methods indicated in Patent Documents 1 to 3 may be used. As the antibody specific to apoB-48, particularly an anti-apoB-48 monoclonal antibody which does not substantially react with apoB-100 is preferably used.

For example, the measurement of apoB-48 can be carried out by, but not limited to, chemiluminescent enzyme immunoassay (CLEIA) based on the principle of the following 2-step sandwich method.

In the measurement method for apoB-48 based on CLEIA, a serum or plasma sample is first treated with a treatment liquid containing a detergent as a major component to make apoB-48 in chylomicrons and chylomicron remnants exposed. Then, this treated sample is added to a suspension of particles on which an anti-apoB-48 monoclonal antibody is immobilized (solid phase; primary antibody) to allow them to react, and the particles are washed and then an alkaline phosphatase-labeled anti-apoB monoclonal antibody (secondary antibody) is added thereto and allowed to react to form, in the sample, a ternary sandwich complex of the primary antibody, apoB-48, and the secondary antibody. Unreacted alkaline phosphatase-labeled antibodies are washed out, and a chemiluminescent substrate (3 -(2' -spiroadamantane)-4-methoxy-(3"-phosphoryloxy)phenyl-1 ,2-dioxethane disodium salt; AMPPD) is added thereto for the enzyme reaction. The amount of apoB-48 bound to the solid phase is reflected by the amount of luminescence associated with the degradation of AMPPD by the enzyme, and, thus, the apoB-48 value can be obtained by measuring the amount of luminescence.

### <Measurement method for TG>

Although any known method can be used for the measurement of the TG value in blood, a measurement method based on an enzyme assay on serum, which is usually widely used in medical institutions, may preferably be used.

### <Measurement method for LDL-C>

Any known methods can be used for the measurement of the LDL-C value. LDL-C is normally calculated using the total cholesterol value (TC) according to the Friedelwald's formula (formula F: (LDL-C) = (TC) - (HDL-C) - (TG/5)). Although any known methods may be used as the measurement methods for the TC value and the HDL-C value, which are usually widely used in medical institutions, cholesterol dehydrogenase-UV method and a direct method, on serum, may preferably be used, respectively. However, when TG is not less than 400 mg/dL, the direct method based on the principle of an enzyme reaction, but not the method using the formula F, may be used because a correct value cannot be calculated with the latter method.

In the method of the present invention, a ratio of the apoB-48 value divided by the TG value and a ratio of the apoB-48 value divided by the LDL-C value are calculated based on the above measured three values. In cases where both of the ratios are higher than the respective cut-off values, the possibility of type III hyperlipidemia can be determined to be high.

Herein, the respective cut-off values can be set preferably based on the ROC (Receiver Operating Characteristic) analysis, more preferably based on the ROC (Receiver Operating Characteristic) analysis on a general population of patients with various types of dyslipidemia. The ROC analysis is an analysis based on an ROC curve obtained by varying the cut-off value and plotting the true positive rates on the vertical axis, and plotting the 1 - true negative rates for the individual cut-off values on the horizontal axis, and is a method in which a cut-off value corresponding to the point on the ROC curve closest to the point where the true positive rate as well as the true negative rate is 1 (the top left corner of the graph) is employed as a cut-off value corresponding to the highest diagnostic efficiency. Herein, "true positive rate" is also referred to as "positive certainty rate," "positive rate," or "sensitivity," and represents a value for the ratio of the number of cases determined as positive relative to the true-positive cases, while "specificity true negative rate" is also referred to as "true negative rate" or "specificity," and represents a value for the ratio of the number of cases determined as disease-free relative to the true-negative cases.

In the Example below, the ROC analysis was performed on 123 human patients with dyslipidemia and the analysis resulted in 0.045 for the cut-off value of the ratio of the apoB-48 value (µg/mL) divided by the TG value (mg/dL) and 0.109 for the cut-off value of the ratio of the apoB-48 value (µg/mL) divided by the LDL-C value (mg/dL). Thus, these values can be used as the respective cut-off values. However, when considering the difference in measurement values depending on measurement methods and/or the difference based on the different general populations, the respective cut-off values are not limited to those values, but usually selected from the range of these values ± 20%, preferably from the range of these values ± 10%.

According to the method of the present invention, such a simple operation as the measurement of the apoB-48 value, TG value and LDL-C value in a blood sample allows the determination of type III hyperlipidemia with high diagnostic efficiency to be achieved. As demonstrated in the Examples below, the diagnostic efficiency was 72% in the method described in Non-Patent Document 5, which uses the ratio of the apoB-48 value divided by the TG value, while the diagnostic efficiency was 89% in the method of the present invention. The "diagnostic efficiency" is also referred to as "proper diagnosis rate" and represents a ratio of the number of properly determined cases to the total test cases.

### Examples

### Subject Patients and Measurement Method

For the sera from 123 human patients with dyslipidemia who had been diagnosed with dyslipidemia according to the criteria in the guidelines (*supra*), the apoB-48 value, the TG value and the LDL-C value were measured. The numbers of cases of type I, type IIa, type IIb, type III, type IV, and type V hyperlipidemia according to the WHO classification were 4, 29, 33, 11, 23, and 23, respectively. The apoB-48 value was measured using the "apoprotein B-48 assay kit" (manufactured by Fujirebio Inc.) and the automated immunoassay system "LUMIPULSE Forte" (manufactured by Fujirebio Inc.). The TG value and the LDL-C value were calculated using the conventional enzyme method and the conventional formula F method, respectively.

### Comparative Example 1

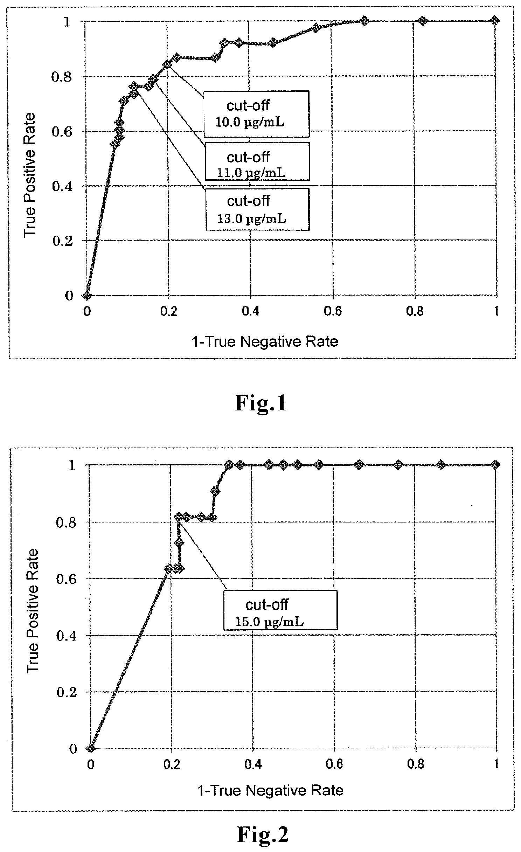

An ROC curve (Receiver Operating Characteristic curve) (Figure 1) was generated for apoB-48 values to compute the cut-off value (10.0 µg/mL) of the apoB-48 value for classifying type I, type III and type V hyperlipidemia as positive, and classifying type IIa, type IIb and type IV hyperlipidemia as negative. Based on the cut-off value, any samples indicating measurements equal to or more than the cut-off value were classified as positive, while any samples indicating measurements less than the cut-off value were classified as negative. The true positive rate in the samples classified as positive, the true negative rate in the samples classified as negative, and the overall diagnostic efficiency were calculated.

### Comparative Example 2

An ROC curve (Receiver Operating Characteristic curve) (Figure 2) was generated for apoB-48 values to compute the cut-off value (15.0 µg/mL) of the apoB-48 value for classifying type III hyperlipidemia as positive, and classifying type I, type IIa, type IIb, type IV and type V hyperlipidemia as negative. Based on the cut-off value, any samples indicating measurements equal to or more than the cut-off value were classified as positive, while any samples indicating measurements less than the cut-off value were classified as negative. The true positive rate in the samples classified as positive, the true negative rate in the samples classified as negative, and the overall diagnostic efficiency were calculated.

### Comparative Example 3

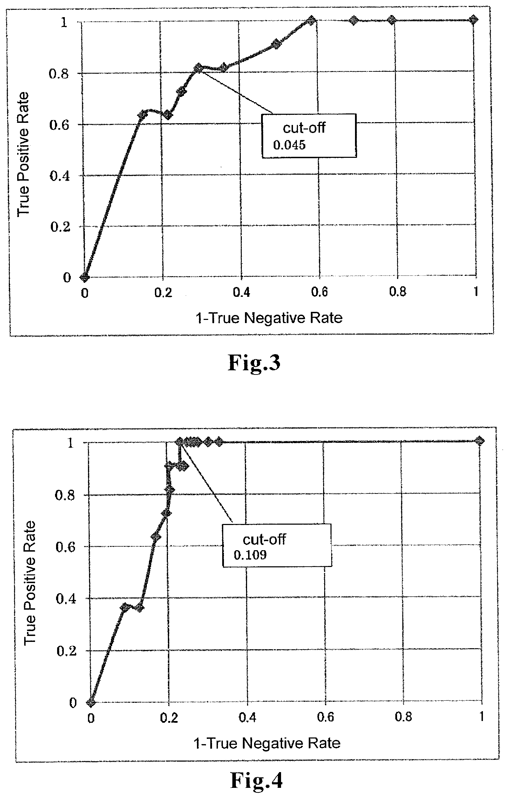

An ROC curve (Figure 3) was generated for ratios of the apoB-48 value divided by the TG value (apoB-48/TG) to compute the cut-off value (0.045) for classifying type III hyperlipidemia as positive, and classifying type I, type IIa, type IIb, type IV and type V hyperlipidemia as negative. Based on the cut-off value, any samples indicating measurements equal to or more than the cut-off value were classified as positive, while any samples indicating measurements less than the cut-off value were classified as negative. The true positive rate in the samples classified as positive, the true negative rate in the samples classified as negative, and the overall diagnostic efficiency were calculated.

### Comparative Example 4

An ROC curve (Figure 4) was generated for ratios of the apoB-48 value divided by the LDL-C value (apoB-48/LDL-C) based on the ROC to compute the cut-off value (0.109) for classifying type III hyperlipidemia as positive, and classifying type I, type IIa, type IIb, type IV and type V hyperlipidemia as negative. Based on the cut-off value, any samples indicating measurements equal to or more than the cut-off value were classified as positive, while any samples indicating measurements less than the cut-off value were classified as negative. The true positive rate in the samples classified as positive, the true negative rate in the samples classified as negative, and the overall diagnostic efficiency were calculated.

### Example 1

Based on the cut-off values (0.045 and 0.109, respectively) of the ratio of apoB-48/TG and the ratio of apoB-48/LDL-C for classifying type III hyperlipidemia as positive, and classifying type I, type IIa, type IIb, type IV and type V hyperlipidemia as negative identified in the respective ROC curves (Figures 3 and 4), each sample was classified as positive or negative, such that any sample indicating measurements equal to or more than the cut-off values with respect to both the ratios was classified as positive, and otherwise as negative. The true positive rate in the samples classified as positive, the true negative rate in the samples classified as negative, and the overall diagnostic efficiency were calculated.

The true positive rates, true negative rates, and diagnostic efficiencies in Comparative Examples 1 to 4 and Example 1 are presented in Table 1. In Comparative Example 1, the diagnostic efficiency for type I, type III and type V hyperlipidemia based on the cut-off which had been identified in the ROC curve of the apoB-48 value was 81%. In Comparative Example 2, the diagnostic efficiency for type III hyperlipidemia based on the cut-off which had been identified in the ROC curve of the apoB-48 value was 77%. In Comparative Example 3, the diagnostic efficiency for type III hyperlipidemia based on the cut-off which had been identified in the ROC curve of the apoB-48/TG value was 72%. In Comparative Example 4, the diagnostic efficiency for type III hyperlipidemia based on the cut-off which had been identified in the ROC curve of the apoB-48/LDL-C value was 79%. In Example 1, the diagnostic efficiency for type III hyperlipidemia based on the cut-off of the apoB-48/TG value and the apoB-48/LDL-C value identified in the ROC curves was indicated to be 89%. By using the apoB-48/TG and the apoB-48/LDL-C, a higher diagnostic efficiency was indicated.

Usually, any test subject has already been measured for the TG value and the LDL-C value when the subject is diagnosed with dyslipidemia. It was found that the method of the present invention provided high diagnostic efficiency for type III hyperlipidemia by measuring the apoB-48 value in the same serum sample used for the measurements of the TG value and the LDL-C value, or in a serum or plasma sample collected at almost the same period of time, and combining the value with the known TG and LDL-C values, specifically, by using the ratios of apoB-48/TG and apoB-48/LDL-C.

**Table 1**

| Item | Example | Positive | Negative | True Positive Rate | True Negative Rate | Diagnostic Efficiency |
|---|---|---|---|---|---|---|
| apoB-48 | Comparative Example 1 | I, III, V | IIa, IIb, IV | 84% | 80% | 81% |
| apoB-48 | Comparative Example 2 | III | I, IIa, IIb, IV, V | 82% | 78% | 77% |
| apoB-48/TG | Comparative Example 3 | III | I, IIa, IIb, IV, V | 82% | 71% | 72% |
| apoB-48/LDL-C | Comparative Example 4 | III | I, IIa, IIb, IV, V | 100% | 77% | 79% |
| apoB-48/TG + apoB-48/LD L-C | Example 1 | III | I, IIa, IIb, IV, V | 82% | 90% | 89% |

## Claims

1. A method of assisting determination of type III hyperlipidemia, the method comprising the steps of:
measuring the apoB-48 value, triglyceride value and LDL-cholesterol value in a blood sample isolated from a living body; and
calculating a ratio of the apoB-48 value divided by the triglyceride value and a ratio of the apoB-48 value divided by the LDL-cholesterol value,
wherein the fact that both the ratio of the apoB-48 value divided by the triglyceride value and the ratio of the apoB-48 value divided by the LDL-cholesterol value are higher than the respective cut-off values indicates that the living body is likely to have type III hyperlipidemia.

2. The method according to claim 1, wherein the blood sample is a serum sample.

3. The method according to claim 1 or 2, wherein said apoB-48 value is measured using an antibody specific to apoB-48.
